# EUROPÄISCHE PATENTANMELDUNG

(11) **EP 0 672 643 A2**
(43) Veröffentlichungstag der Anmeldung: **20.09.1995**
(21) Anmeldenummer: 95103156.6
(22) Anmeldetag: 06.03.1995
(51) Int. Cl.: C07C 29/141

(54) **Hydrogenolytische Reduktion eines Aldehyds zu einem Alkohol in Gegenwart eines Monolithkatalysators**

(30) Priorität: 15.03.1994 AT 541/94
(71) Anmelder: Chemie Linz Gesellschaft m.b.H., A-4021 Linz (AT)
(72) Erfinder: Kos, Carlo, Dr., A-4060 Leonding (AT); Winetzhammer, Willibald, Dipl.-Ing. Dr., A-4641 Steinhaus (AT); Pollhammer, Stefan, A-4030 Linz (AT); Schaller, Josef, A-4020 Linz (AT); Hebesberger, Friedrich, A-4073 Wilhering (AT); Lust, Ernst, A-4020 Linz (AT); Haar, Robert, A-4210 Engerwitzdorf (AT)
(74) Vertreter: Kunz, Ekkehard, Dr.

(57) **Zusammenfassung**

Verfahren zur hydrogenolytischen Reduzierung eines Aldehyds oder dessen Halbacetals oder dessen Vollacetals in Gegenwart eines inerten organischen Verdünnungsmittels und eines Monolithkatalysators bei Wasserstoffdrücken von 0,01 bis 7,0 MPa und bei Temperaturen von -10 bis 200 °C.

## Beschreibung

Es ist seit langem bekannt, daß Aldehyde mit Wasserstoff katalytisch zu Alkoholen umgesetzt werden können. So ist etwa in US 4,451,677 eine mehrstufige Hydrogenolyse von Aldehyden zu den entsprechenden Alkoholen in Gasphase beschrieben. Reaktionen in der Gasphase erfordern aber aufwendige Apparaturen. Aus US 2,434,110 ist ein Verfahren zur Hydratisierung und anschließender Hydrogenolyse von alpha-ungesättigten Aldehyden in Flüssigphase bekannt, wobei in der Hydratisierungsstufe Wasser an die Doppelbindung addiert wird, sodaß eine Verbindung, die eine Hydroxy- und eine Aldehydgruppe enthält, erhalten wird, die in der Hydrogenolysestufe zur entsprechenden Dihydroxyverbindung reduziert wird. Zur Hydrogenolyse wird der Katalysator in die hydratisierte Reaktionslösung gegeben, sodaß eine Katalysatorsuspension entsteht. Ebenfalls in einer Katalysatorsuspension werden gemäß EP-A1-0 219 905 alpha,beta-ungesättige Aldehyde zu den entsprechenden alpha,beta-ungesättigten Alkohlen mit Wasserstoff in Gegenwart eines Alkalimetallhydroxids oder -alkoxids umgesetzt.

Aus Katalysatorsuspensionen muß aber der Katalysator nach erfolgter Umsetzung aufwendig abgetrennt werden, wobei wasserstoffbeladene Katalysatoren, die mit Luftsauerstoff in Kontakt geraten, oft Ursache von Bränden sind.

In GB 784,359 wurde die Umsetzung einer Mischung von Aldehyden und Olefinen mit Wasserstoff in Gegenwart von Katalysatorpellets vorgeschlagen, wobei die Gegenwart von Wasser als wesentlich beschrieben wird. Unter diesen Bedingungen soll die Hydrierung der olefinischen Doppelbindungen unterbunden werden, während die Aldehydgruppe zur entsprechenden Alkoholgruppe reduziert wird.

Gemäß EP-A1-0 535 565 wird der Katalysator bei der Umsetzung von Hydroxypropionaldehyd mit Wasserstoff zur Herstellung von 1,3-Propandiol in Form eines Festbettes, über den die Reaktionslösung rieselt, angelegt. Ähnlich wird die katalystische Hydrogenolyse von unter anderem Aldehyden zur Herstellung entsprechender Alkohole mit Hilfe von Wasserstoff gemäß WO 87/07598, WO 88/05767 oder EP-A1-0 319 208 durchgeführt. Festbett- oder Rieselbettkatalysatoren haben aber den Nachteil, daß die Reduktion diffusionskontrolliert verläuft und daß deshalb eine lange Kontaktzeit des Katalysators mit der Reaktionsmischung in Kauf genommen werden muß.

Es wurde nun unerwarteterweise gefunden, daß bei der Reduktion von Aldehyden zu Alkoholen mit Hilfe von Wasserstoff in flüssiger Phase an einem Monolithträgerkatalysator nur kurze Kontaktzeiten nötig sind und daß die Reaktionsgeschwindigkeit der Reduktion daher erheblich gesteigert werden kann.

Gegenstand der Erfindung ist daher ein Verfahren zur Steigerung der Reaktionsgeschwindigkeit der katalysierten Hydrogenolyse von Aldehyden mit Wasserstoff, das dadurch gekennzeichnet ist, daß als Katalysator ein Monolithkatalysator eingesetzt wird.

Unter Aldehyd ist ein Aldehyd selbst, dessen Halbacetal oder dessen Vollacetal zu verstehen. Der Aldehyd kann dabei sowohl ein aliphatischer, als auch ein aromatischer Aldehyd mit jeweils einer oder mehreren Aldehydgruppen bzw. Halbacetal- oder Acetalgruppen sein. Ein aliphatischer Aldehyd ist ein geradkettiger, verzweigter oder cyclischer Mono-, Di-, oder Mehrfachaldehyd, der bevorzugt 1 bis 22 C-Atome, besonders bevorzugt 1 bis 16 C-Atome aufweist, wobei die Alkylketten unsubstituiert oder durch unter den Reaktionsbedingungen inerte Gruppen substituiert sein können. Solche inerten Gruppen sind beispielsweise Arylgruppen, wie unsubstituiertes oder durch Alkyl, bevorzugt mit 1 bis 6, besonders bevorzugt mit 1 bis 4 C-Atomen, wie Methyl, Ethyl, iso-Propyl, Butyl, neo-Pentyl, Hexyl, Alkoxy, bevorzugt mit 1 bis 6, besonders bevorzugt mit 1 bis 4 C-Atomen, wie Methoxy-, Ethoxy-, Butoxy-, iso Pentoxy-, Hexoxygruppen oder Halogen substituiertes Phenyl oder Naphthyl oder Alkoxygruppen, bevorzugt solche mit 1 bis 6 C-Atomen. Aromatische Aldehyde sind Aldehyde, bei denen eine oder mehrere Aldehydgruppen direkt an ein aromatisches C-Atom gebunden sind, beispielsweise in einer Phenyl-, Naphthyl-, oder Pyridingruppe wie Benzaldehyd, Phthalaldehyd, Naphthylaldehyde, Pyridinaldehyd oder -dialdehyd. Bevorzugt werden unsubstituierte, aliphatische Dialdehyde mit 1 bis 22 C-Atomen eingesetzt.

Zur Herstellung von Aldehyden sind eine Vielzahl von Verfahren bekannt. Gemäß US-4,607,126 oder US 4,769,464 können Aldehyde und Dialdehyde in technischem Maßstab auf bequeme Weise beispielsweise durch Ozonolyse und Reduktion olefinischer Doppelbindungen hergestellt werden. Dabei entstehen die Aldehyde je nach den pH-Wert Bedingungen als Aldehyde, deren Halbacetale oder deren Vollacetale. Aldehyde, die gemäß einer der genannten Patentschriften herstellbar sind, werden bevorzugt in das erfindungsgemäße Verfahren eingesetzt.

Maßgeblich im erfindungsgemäßen Verfahren ist, daß der Aldehyd in einem Verdünnungsmittel zumindest teilweise gelöst vorliegt. Unter Verdünnungsmittel sind dabei übliche, bei der Hydrogenolyse verwendete Verdünnungsmittel zu verstehen, beispielsweise aliphatische oder aromatische, gegebenenfalls chlorierte Kohlenwasserstoffe, wie Pentan, Hexan, Cyclohexan, Toluol, Xylole, Methylenchlorid, Dichlorethan, Chlorbenzole, Carbonsäureester wie Essigsäuremethyl-, -ethyl oder -butylester, Ether wie Diethylether, Diisopropylether, Tetrahydrofuran, Ketone wie Aceton, Methylbutylketon, Alkohole wie Methanol, Ethanol, iso-Propanol, Butanol, Hexanol, Wasser oder Mischungen solcher Verdünnungsmittel. Bevorzugt werden aliphatische Alkohole mit 1 bis 6 C-Atomen als Verdünnungsmittel oder Mischungen solcher Alkohole mit Wasser eingesetzt.
Die Konzentration der Aldehyde in der Lösung ist für das erfindungsgemäße Verfahren nur von geringer Bedeutung. Im allgemeinen werden möglichst hohe Aldehydkonzentrationen hergestellt, um Verdünnungsmittel zu sparen, wobei die Konzentration aber nicht so hoch sein soll, daß die Aldehyde aus der Reaktionslösung ausfallen.

Unter einem Monolithkatalysator ist ein Katalysator zu verstehen, der aus einem Träger, der mit einem wirksamen Katalysatorgrundstoff beschichtet ist, besteht. Der Träger besitzt bevorzugt eine möglichst große Oberfläche, die beispielsweise durch waben- oder lamellenförmige Strukturierung erreicht werden kann. Der Träger liegt in einem Stück vor und kann aus dazu geeigneten Materialien, beispielsweise aus Metall, Glas, Keramik, Kunststoff bestehen. Bevorzugt ist ein Metallträger, beispielsweise aus Stahl, Aluminium, da dieser die Reaktionswärme aufnehmen und wieder in das umgehende Reaktionsmedium abgeben kann. Es hat sich nämlich herausgestellt, daß es bei Verwendung nichtleitender Materialien als Träger zu lokalen Überhitzungen im Reaktionsmedium kommen kann, sodaß Ausbeuten und Reinheit der Reaktionsprodukte negativ beeinflußt werden können.

Als wirksamer Katalysatorgrundstoff sind bei der Reduktion von Aldehyd oder dessen Halbacetal oder dessen Vollacetal zu Alkoholen übliche Katalysatorgrundstoffe zu verstehen. Übliche Katalysatorgrundstoffe sind hydrierwirksame Metalle, beispielsweise Titan, Vanadium, Chrom, Molybdän, Wolfram, Eisen, Osmium, Kobalt, Rhodium, Iridium, Nickel, Palladium, Platin, Kupfer, Silber, Zink, deren Oxide, oder Mischungen solcher Metalle oder Metalloxide. Bevorzugt werden im erfindungsgemäßen Verfahren Edelmetalle als Katalysatorgrundstoff eingesetzt. Die Ausbeuten sind beim erfindungsmäßen Verfahren an sich von der eingesetzten Menge des Katalysatorgrundstoffes unabhängig, jedoch empfiehlt es sich zur Erzielung einer ausreichenden Hydrierungsgeschwindigkeit 0,05 bis 5,0 Gew.% bevorzugt 0,1 bis 2,0 Gew.% Katalysatorgrundstoff bezogen auf die Gesamtmenge an Lösung des Aldehyds vorzulegen.
Zur Fixierung des Katalysatorgrundstoffes auf dem Träger kann der Träger mit einer katalysatorenwirksamen Schicht beschichtet sein, beispielsweise mit einem Aluminium/Siliciumoxid-coating, auf die der wirksame Katalysatorgrundstoff aufgebracht ist.

Die Herstellung solcher Katalysatoren kann durch ein übliches Beschichtungsverfahren, beispielsweise durch Aufdampfen des Katalysatorgrundstoffes auf einen Träger oder Imprägnierung des Trägers mit dem Katalysatorgrundstoff erfolgen.

Zur Durchführung des erfindungsgemäßen Verfahrens wird die Lösung des Aldehyds im verwendeten Verdünnungsmittel mit dem Monolithkatalysator und mit Wasserstoff in Kontakt gebracht. Dabei kann der Monolithkatalysator in das, den Aldehyd enthaltende, Verdünnungsmittel eingebracht und Wasserstoff unter Rühren zugesetzt werden, oder das, den Aldehyd enthaltende Verdünnungsmittel wird zusammen mit Wasserstoff kontinuierlich über den Monolithkatalysator geleitet. Der Aldehyd kann aber auch als solcher oder gelöst in einem Verdünnungsmittel in einen Reaktor, in dem Wasserstoff, Monolithkatalysator und Verdünnungsmittel vorgelegt sind, eingebracht werden.

Die Reduktion, die im allgemeinen exotherm verläuft, wird bei Temperaturen von etwa -10 bis 200 °C, bevorzugt von etwa 15 bis 150 °C, besonders bevorzugt von etwa Raumtemperatur bis 100 °C ausgeführt. Dabei wird der Wasserstoff durch Einleiten oder Aufdrücken wie üblich zugesetzt, wobei das Wasserstoffgas gegebenenfalls Anteile von Inertgasen wie Stickstoff, Argon, Helium enthalten kann. Der Wasserstoffdruck beträgt im allgemeinen 0,01 bis 7,0 MPa, bevorzugt 0,1 bis 3,0 MPa. Der jeweils geeignete Wasserstoffdruck und die jeweils geeignete Temperatur sind dabei für den jeweiligen Aldehyd leicht durch einen Vorversuch zu ermitteln. Pro Mol Aldehyd-, Hemiacetal- oder Vollacetalgruppe muß dabei mindestens die stöchiometrische Menge an Wasserstoff eingesetzt werden.

Bei der Reduktion entsteht der dem eingesetzten Aldehyd entsprechende Alkohol. Zur Aufarbeitung wird die hydrierte Produktlösung vom Katalysator, etwa durch Herausnehmen des Katalysatorträgers aus dem Reaktionsgemisch oder durch Abpumpen der Produktlösung aus dem Reaktionsgefäß, das den Katalysator enthält, auf einfache Weise abgetrennt. Eine komplizierte Abtrennung der Katalysatorsuspension durch Filtrieren oder Abzentrifugieren, wie bisher erforderlich, entfällt. Der entstandene Aldehyd wird aus der hydrierten Reaktionsmischung durch Entfernen des Verdünnungsmittels und des gebildeten Reaktionswassers isoliert und gegebenenfalls nach üblichen Verfahren wie Kristallisieren, Chromatographie- oder Destillationsverfahren gereinigt.

In einer bevorzugten Ausführungsform der Erfindung wird ein Monolithkatalysator, der mit einem Edelmetall als wirksamer Katalysatorgrundstoff beschichtet ist, in einen Umwälzreaktor vorgelegt. Der Reaktor wird mit einer Mischung aus aliphatischen Alkohol mit 1 bis 6 C-Atomen und Wasser als Verdünnungsmittel beschickt. Die Mischung wird auf Temperaturen von 40 bis 100 °C erhitzt und Wasserstoff aufgedrückt, sodaß ein Druck von etwa 1 bis 3 MPa aufrechterhalten wird.
In diese Mischung wird der Aldehyd unverdünnt zudosiert und reagieren gelassen. Nach erfolgter Umsetzung, die gaschromatographisch verfolgt wird, wird das Verdünnungsmittel aus dem Reaktionsgemisch entfernt und der Rückstand gegebenenfalls destillativ oder chromatographisch gereinigt.

Auf die erfindungsgemäße Art und Weise werden Aldehyde auf einfache Art und Weise in kurzer Reaktionszeit zu sehr reinen Alkoholen in hohen Ausbeuten reduziert. Die Erfindung stellt daher eine Bereicherung der Technik dar.

### Beispiel 1

### Herstellung von 1,8-Octandiol

In einem Umwälzreaktor der mit 7 monolithischen, zylindrischen Katalysatorkörpern (Metallträger beschichtet mit einem Aluminium/Siliciumoxidcoating, auf dem ein Pt-Katalysator aufgebracht ist) mit jeweils 92 mm Durchmesser und einer Höhe von 78 mm bestückt ist, wurden innerhalb von 30 Minuten 1.420 g (10,0 mol) Octandial mit einer Reinheit von 97,7 % (GC) in ein Gemisch aus 7.200 g Methanol und 800 g Wasser, bei einer Temperatur von 80 - 85 °C und einem Wasserstoffgesamtdruck von 15 bar, zudosiert und hydrogenolytisch zum 1,8-Octandiol reduziert bis keine Wasserstoffaufnahme mehr feststellbar war.

Die Reaktion wurde mittels GC-Analytik verfolgt und bei einem Gehalt von < 1 % 8-Hydroxyoctanaldehyd abgebrochen. Der Wasserstoffverbrauch betrug 445 Normliter, der Umsatz betrug 99 % mit einer Reinheit von 96,8 % GC. Die Reaktionslösung wurde nach der Reaktion durch Destillation vom Lösungsmittel und Wasser befreit, der Rückstand wurde durch Vakuumdestillation bei einer Temperatur von 130 °C und einem Druck von 8 mbar gereinigt.
Schmelzpunkt 58 - 60 °C.

### Beispiel 2

### Herstellung von 1,12-Dodecandiol

226 g (1,14 mol) Dodecandial mit einer Reinheit von 99,7 % (GC) wurden analog Beispiel 1 zum 1,12-Dodecandiol reduziert, bis keine Wasserstoffaufnahme mehr feststellbar war.
Die Reaktion wurde mittels GC-Analytik verfolgt und bei einem Gehalt von < 1,6 % 12-Hydroxydodecanaldehyd abgebrochen. Der Wasserstoffverbrauch betrug 51 Normliter, der Umsatz 97 %, mit einer Reinheit von 96,7 % GC. Die Reaktionslösung wurde nach der Reaktion durch Destillation vom Lösungsmittel und Wasser befreit, der Rückstand wurde durch Vakuumdestillation bei einer Temperatur von 189 °C und einem Druck von 12 mbar gereinigt.
Schmelzpunkt 80 - 81 °C.

### Beispiel 3

### Herstellung von n-Nonanol

Analog Beispiel 1 wurden 800 g (5,6 mol) Nonanal mit einer Reinheit von 99,7 % (GC) hydrogenolytisch zum n-Nonanol reduziert bis keine Wasserstoffaufnahme mehr feststellbar war.
Die Reaktion wurde mittels GC-Analytik verfolgt und bei einem Gehalt von < 1 % Nonanaldehyd abgebrochen. Der Wasserstoffverbrauch betrug 124 Normliter, der Umsatz betrug 98,9 % mit einer Reinheit von 98,2 % GC. Die Reaktionslösung wurde nach der Reaktion durch Destillation vom Lösungsmittel und Wasser befreit. Der Rückstand wurde durch Vakuumdestillation bei einer Temperatur von 100 °C und einem Druck von 27 mbar gereinigt.
Schmelzpunkt - 5 °C.

### Vergleichsbeispiel 1

142,2 g (1,0 mol) Octandial wurden bei einer Temperatur von 80 °C und einem Druck von 60 bar in einem Gemisch von 450 g Methanol und 50 g Wasser mittels eines herkömmlichen Schlämmkatalysators (Nickelkatalysator auf Aluminium/Siliciumoxid Mischträger Ni 6458 P der Firma Engelhardt) hydrogenolytisch zum 1,8-Octandiol reduziert.
Die Reaktion wurde mittels GC-Analytik verfolgt und bei einem Gehalt von < 2 % 8-Hydroxyoctanaldehyd abgebrochen. Der Wasserstoffverbrauch betrug ca. 44 Normliter, der Umsatz 98 % mit einer Reinheit von ca. 95,8 % GC. Die Reaktionslösung wurde nach der Reaktion vom Katalysator filtriert. Die grünliche Reaktionslösung enthält bis zu 500 ppm Nickelkomplexe und wurde durch Destillation vom Lösungsmittel und Wasser befreit. Der Rückstand wurde durch Vakuumdestillation bei einer Temperatur von 130 °C und einem Druck von 8 mbar gereinigt.
Schmelzpunkt 58 - 60 °C.

### Vergleichsbeispiel 2

142,2 g (1,0 mol) Octandial wurden bei einer Temperatur von 80 °C und einem Druck von 40 bar in einem Gemisch von 450 g Methanol und 50 g Wasser mittels Pt nach Adams (aktiviert durch Hydrierung unter Reaktionsbedingungen) hydrogenolytisch zum 1,8-Octandiol reduziert.
Die Reaktion wurde mittels GC-Analytik verfolgt. Die Reaktion kam nach einem Umsatz von ca. 30 - 35 % zum Erliegen. Erneute Zugabe von frisch aktivierten Katalysator erhöhte den Umsatz aliquot der zugesetzten Katalysatormenge. Mehrere Versuche eine vollständige Hydrierung zu erreichen schlugen fehl.
Der Wasserstoffverbrauch betrug ca. 14,7 Normliter, der Umsatz 33 % mit einer Reinheit von ca. 35,8 % GC. Die Reaktionslösung wurde nach der Reaktion vom Katalysator filtriert und durch Destillation vom Lösungsmittel und Wasser befreit. Der Rückstand wurde durch Vakuumdestillation bei einer Temperatur von 130 °C und einem Druck von 8 mbar gereinigt. Der Destillationssumpf polymerisierte bei einer Destillationssumpftemperatur über 220 °C.
48 g (33 % Ausbeute d. Th.) Produkt mit einem Schmelzpunkt 58 - 60 °C.

## Patentansprüche

1. Verfahren zur hydrogenolytischen Reduktion eines Aldehyds zu einem Alkohol in einem Verdünnungsmittel in Gegenwart eines Katalysators der geeignet ist, eine hydrogenolytische Reduktion eines Aldehyds zu einem Alkohol zu katalysieren, dadurch gekennzeichnet, daß die Reduktion in Gegenwart eines Monolithkatalysators bei einem Wasserstoffdruck von 0,01 bis 7,0 MPa und bei Temperaturen von -10 bis 200 °C erfolgt, worauf der gebildete Alkohol aus dem Reaktionsgemisch isoliert wird.

2. Verfahren nach Anspruch 1, dadurch gekennzeichnet, daß der Monolithkatalysator aus einem Katalysatorträger, der mit einem Edelmetall beschichtet ist, besteht.

3. Verfahren nach Anspruch 2, dadurch gekennzeichnet, daß der Katalysatorträger aus einem Metall besteht.

4. Verfahren nach einem der Ansprüche 2 oder 3, dadurch gekennzeichnet, daß der Katalysatorträger mit einer katalysatorwirksamen Beschichtung zur Fixierung der katalysatorwirksamen Substanz versehen ist.

5. Verfahren nach einem der Ansprüche 1 bis 4, dadurch gekennzeichnet, daß ein Wasserstoffdruck von 0,1 bis 3,0 MPa aufrechterhalten wird.

6. Verfahren nach einem der Ansprüche 1 bis 5, dadurch gekennzeichnet, daß eine Temperatur von Raumtemperatur bis 100 °C aufrechterhalten wird.

7. Verfahren nach einem der Ansprüche 1 bis 6, dadurch gekennzeichnet, daß der Aldehyd ein aliphatischer Aldehyd ist.

8. Verfahren nach einem der Ansprüche 1 bis 7, dadurch gekennzeichnet, daß der Aldehyd ein aliphatischer Dialdehyd ist.

9. Verfahren nach einem der Ansprüche 1 bis 8, dadurch gekennzeichnet, daß der Aldehyd ein unsubstituierter, aliphatischer Dialdehyd mit 1 bis 22 C-Atomen ist.
